# DEMANDE DE BREVET EUROPEEN

(11) **EP 1 908 452 A1**
(43) Date de publication de la demande: **09.04.2008**
(21) Numéro de dépôt: 07115224.3
(22) Date de dépôt: 29.08.2007
(51) Int. Cl.: A61K 8/19, A61K 8/35, A61K 8/60, A61K 8/97, A61Q 19/04

(54) **Composition pour la peau à effet bonne mine**

(30) Priorité: 03.10.2006 FR 0654058
(71) Demandeur: L'Oréal, 75008 Paris (FR)
(72) Inventeur: Chevalier, Véronique, 94440 Villecresnes (FR)
(74) Mandataire: Rasson, Catherine

(57) **Abrégé**

L'invention concerne une composition comprenant des pigments encapsulés et au moins un agent de coloration de la peau choisi parmi les agents autobronzants, les activateurs de mélanogénèse et leurs mélanges.

La composition de l'invention constitue notamment une composition cosmétique qui donne un effet immédiat de bonne mine, qui se prolonge dans le temps.

## Description

L'invention concerne des compositions à usage topique, destinées à donner un effet de bonne mine à la peau, comprenant au moins un agent de coloration de la peau (autobronzant et/ou activateur de mélanogénèse), et des pigments encapsulés.

L'invention porte également sur l'utilisation de la dite composition pour donner un effet de bonne mine à la peau et bronzer et/ou brunir artificiellement la peau.

De nos jours, il est important d'avoir bonne mine, et une peau hâlée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais qui en contrepartie sont susceptibles d'induire une altération de celle-ci, notamment dans le cas d'une peau sensible ou d'une peau continuellement exposée au rayonnement solaire. Il est donc souhaitable de trouver une alternative au bronzage naturel, qui soit compatible avec les exigences de telles peaux.

La plupart des produits cosmétiques destinés au bronzage artificiel de la peau sont à base de composés mono- ou poly-carbonylés permettant, par interaction avec les acides aminés de la peau, la formation de produits qui vont colorer la peau. Dans ce but, on peut aussi utiliser des activateurs de mélanogénèse.

Ainsi, on sait que la dihydroxyacétone ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration. De plus, la coloration produite sur la peau par la DHA est souvent jugée trop jaune par les utilisateurs.

Ainsi, on est toujours à la recherche de nouveaux composés et de nouvelles compositions permettant de conférer artificiellement à la peau, une coloration lui donnant un effet de bonne mine, proche du bronzage naturel, d'une manière simple, efficace, rapide et sans risque.

Certes, il est connu d'utiliser des produits de maquillage pour donner un peu de couleur à la peau. L'addition de DHA pourrait renforcer ou prolonger la coloration donnée par le maquillage. Toutefois, les produits de maquillage sont colorés grâce à la présence des pigments, et il est difficile d'utiliser des produits de maquillage contenant de la DHA du fait de la présence de ces pigments, notamment des oxydes de fer, incompatibles avec la DHA entraînant déstabilisation de la composition Ainsi, on préfère utiliser la DHA dans des produits blancs.

Il subsiste donc le besoin d'une composition qui ne soit pas une composition colorée mais qui, après application sur la peau, développe une couleur immédiatement et à long terme.

La demanderesse a maintenant découvert la possibilité d'obtenir de telles compositions en associant des pigments encapsulés à un agent de coloration de la peau, tel qu'un autobronzant comme la DHA, et/ou un composé activateur de la mélanogénèse.

La composition selon l'invention permet d'obtenir une coloration de la peau, qui se produit immédiatement à l'application de la composition sur la peau et qui, en outre, dure dans le temps. En outre, l'utilisation d'une telle composition présente l'avantage d'éviter totalement les risques de réaction cutanée généralement attachés aux expositions prolongées précitées (érythèmes, brûlures, perte d'élasticité, apparition de rides, vieillissement prématuré de la peau, et autres).

L'invention a donc pour objet une composition comprenant, des pigments encapsulés et au moins un agent de coloration de la peau choisi parmi les agents autobronzants, les activateurs de mélanogénèse et leurs mélanges.

La composition revendiquée est destinée à une application topique. On entend ici par « application topique » une application externe sur les matières kératiniques, et notamment la peau. La composition étant destinée à une application topique, elle comprend un milieu physiologiquement acceptable. On entend par « milieu physiologiquement acceptable », un milieu compatible avec les matières kératiniques, c'est-à-dire la peau, les lèvres, le cuir chevelu, les cils, les yeux, les ongles et/ou les cheveux. La composition peut constituer notamment une composition cosmétique ou dermatologique.

On entend dans la présente demande, par « pigments encapsulés » des pigments qui sont contenus dans des capsules constituant une coque autour du ou des pigments. Les capsules peuvent contenir une seule sorte de pigments et donc une seule couleur, ou plusieurs sortes de pigments.

Ces pigments encapsulés présentent l'avantage d'être invisibles dans la composition grâce à leur encapsulation, tout en étant libérés facilement de leurs capsules lors de l'application sur la peau. La pression exercée lors de l'application de la composition sur la peau casse les capsules, et le ou les pigments sont libérés, conférant de la couleur à la peau.

Les pigments encapsulés sont bien différents des pigments enrobés couramment utilisés dans les produits de maquillage. En effet, alors que les pigments enrobés comportent un enrobage chimique visant à améliorer sa dispersion dans la composition le contenant, enrobage qui est plus ou moins homogène et qui n'empêche pas la coloration de la composition le contenant, les pigments encapsulés comportent une couche physique constituée par la capsule, cette couche étant bien homogène et isolant de manière étanche le pigment encapsulé de la composition le contenant, de sorte que chaque pigment encapsulé est bien individualisé dans la composition ce qui n'est pas le cas des pigments enrobés.

Les pigments qui sont encapsulés peuvent être choisis parmi les pigments minéraux et les pigments organiques, interférentiels ou non, traités en surface ou non traités, et leurs mélanges. On appelle « pigment », toute matière colorante insoluble dans les matières premières cosmétiques connues de l'homme de l'art. Ils peuvent être seuls (une seule couleur) ou en mélange. Les pigments peuvent être de toute couleur, notamment jaunes, noirs, rouges, bruns, bleus et violets.

On peut citer notamment, comme pigments minéraux, le dioxyde de titane, les oxydes de zirconium, les oxydes de cérium, les oxydes de zinc, les oxydes de fer (noir, jaune ou rouge), les oxydes de chrome, le violet de manganèse, le bleu outremer, l'hydrate de chrome et le bleu ferrique. Tous ces pigments peuvent être éventuellement traités en surface.

Parmi les pigments organiques, on peut citer le noir de carbone, les pigments de type laques organiques de baryum, strontium, calcium ou aluminium dont celles soumises à une certification par la Food and Drug Administration (FDA) (exemples D&C ou FD&C) et ceux exempts de la certification FDA comme les laques à base de carmin de cochenille.

Les pigments sont encapsulés dans des capsules qui doivent être à la fois souples et résistantes. Ainsi, elles doivent être résistantes aux autres matières premières présentes dans la composition mais assez souples pour pouvoir se rompre sous cisaillement lors de l'application sur la peau pour ainsi délivrer la couleur espérée.

Ces capsules peuvent être par exemple en un matériau qui peut être choisi parmi:
- les esters de jojoba,
- les polymères ou copolymères d'acide acrylique et/ou d'acide méthacrylique,
- les dérivés de cellulose comme l'hydroxypropyl methylcellulose,
- et leurs mélanges.

Comme pigments encapsulés par des capsules en esters de jojoba, on peut citer par exemple les sphères commercialisées par la société Floratech (« Spheres of jojoba esters ») sous les dénominations Florasomes et décrites dans le document US-A-6,432,428.

Comme pigments encapsulés par des capsules en polymères ou copolymères d'acide acrylique et/ou d'acide méthacrylique, on peut citer par exemple les microcapsules à base de copolymère acrylates/ammonium methacrylate, commercialisées par la société Tagra et décrites dans le document WO-A-01/35933.

Comme pigments encapsulés par des capsules en dérivés de cellulose, on peut citer par exemple les sphères comprenant du mannitol, de la cellulose, de l'hydroxypropyl methylcellulose, commercialisées par la société Induchem.

On peut utiliser aussi dans la même composition plusieurs sortes de capsules contenant des pigments encapsulés et faites en des matériaux différents d'encapsulation.

Les capsules peuvent, outre les pigments, contenir des additifs tels que par exemple des agents plastifiants et/ou des agents opacifiants afin que la couleur des pigments encapsulés soit encore mieux dissiimulée.

La taille de ces capsules, c'est-à-dire leur diamètre moyen en nombre, peut aller par exemple de 20 à 700 µm.

Le pourcentage de pigments par rapport au poids total du pigment encapsulé (poids du pigment encapsulé = poids de la capsule + poids des pigments) peut varier dans une large mesure. La quantité de pigments peut aller par exemple de 10 à 80 % en poids, de préférence de 20 à 60 % en poids et mieux de 20 à 50 % en poids par rapport au poids total du pigment encapsulé.

La composition selon l'invention peut contenir une quantité de pigments encapsulés (poids de la capsule + poids des pigments) allant par exemple de 0,01 à 20 % en poids, de préférence de 0,05 à 15 % en poids et mieux de 0,5 à 10 % en poids par rapport au poids total de la composition.

A côté des pigments encapsulés, la composition selon l'invention contient un agent de coloration de la peau choisi parmi les agents autobronzants, les activateurs de mélanogénèse et leurs mélanges. Ces agents vont lentement colorer la peau.

La quantité d'agent(s) de coloration de la peau dans les compositions de l'invention peut aller par exemple de 0,05 à 15 % en poids, de préférence de 0,1 à 10 % en poids, et mieux de 0,5 à 10 % en poids par rapport au poids total de la composition.

Comme agents autobronzants, on peut citer notamment les composés mono- ou poly-carbonylés tels que la dihydroxyacétone (DHA), l'isatine, l'alloxane, la ninhydrine, le glycéraldéhyde, l'aldéhyde mésotartrique, la glutaraldéhyde, l'érythrulose, les dérivés de pyrazolin-4,5-diones, tels que décrits dans les documents FR-A-2466492 et WO-A-97/35842, les dérivés de 4,4-dihydroxypyrazolin-5-ones tels que décrits dans le document EP-A-0903342, et leurs mélanges.

Dans un mode de réalisation particulièrement préféré de l'invention, on utilise plus particulièrement comme agents autobronzants, la dihydroxyacétone (DHA), l'érythrulose, et leurs mélanges.

On peut aussi utiliser des mélanges de composés, mélanges qui peuvent mimer l'activité de la polyphénol oxydase. Ainsi, en présence d'hydrogénocarbonate de sodium et d'un sel métallique dans un rapport de 1/50 à 1/200 avec un rapport optimal à 1/100, les polyphénols vont, par oxydation, donner une couleur marron à la peau ou aux cheveux. On peut donc utiliser un mélange comprenant un polyphénol, de l'hydrogénocarbonate et un sel métallique.

Comme activateurs biologiques de la mélanogénèse, on peut citer par exemple :
1) Les composés qui stimulent la synthèse de la mélanine, comme :
   a) Certains extraits de plantes tels que les extraits de rosacée notamment du *genre Sanguisorba officinalis,* tels que décrits dans le document EP-A-993826, et les extraits de chrysanthème *(chrysanthemum)* tels que décrits dans le document EP-A-1014934, les catéchines et les esters d'acide gallique de catéchines ainsi que les extraits de thé vert en contenant, comme décrits dans le document WO-99/66897 ;
   b) Les diols aliphatiques ou cycliques comme décrits dans l'article de Brown DA et al, J. Invest. Dermatol., 110, 4, 428-437, 1998 ;
   c) Les peptides de points isoélectriques compris entre 6 à 11, comme décrits dans le document WO-A-99/37279 ;
   d) Les complexes nucléopeptidiques, comme décrits dans le document WO-A-98/12212 ;
   e) Les antagonistes de récepteur adénosine-1 ou les agonistes de récepteur adénosine-2, comme décrits dans le document WO-A-98/15276 ;
   f) Les complexes d'ions métalliques et de peptones, comme décrits dans le document US-A-5,698,184 ;
   g) Les alpha-hydroxyacides et leurs dérivés (par exemple esters), les alpha-cétoacides et leurs dérivés (par exemple esters), les béta-hydroxyacides et leurs dérivés (par exemple esters), les rétinoïdes comme la vitamine A (rétinol), les acides comme l'acide trichloroacétique et l'acide trifluoroacétique, les phénols et la methoxypropyl gluconamide, comme décrits dans le document WO-A-99/51198 ;
   h) les composés qui stimulent la synthèse de la mélanine par utilisation de substrats selon la voie de synthèse : L- Tyrosine, L-dihydrophényl alanine et L-dopa [voir Smit N.P.M. et al J. Invest. Dermatol., 109, 796-800, 1997].
2) Les composés qui stimulent l'activité ou l'expression de la tyrosinase, comme :
   a) L'extrait de Caesapinia sappan L. décrit dans le document EP-A-0820761;
   b) Les extraits de Parameria laevigata, Piper cubeba, Sonchus arvensis L., Pluchea indica L., Massoia aromatica Becc., Alstonia scholaris, Alycia reindwartii BI., Cinnamomum sintoc BI, Arctostaphylos, Chenopodium, Poterium, Gautheria, comme décrits dans le document EP-A-0914816 ;
   c) Les prostaglandines, leurs dérivés, sels et analogues, comme décrits dans le document WO-A-98/00100 ;
   d) Les activateurs de NO/cGMP/protein kinase G, comme décrits dans le document WO-A-98/11882 ;
   e) Les composés qui stimulent l'activité ou l'expression de la tyrosinase par augmentation du taux d'AMPc intra cellulaire, comme :
      - Les peptides pro-opiomélanocortiques, comme décrits dans le document WO-A-98/25584 ;
      - Les analogues de l'AMPc [voir Mc Lane et al, Biochem. Biophys. Res. Comm, 145, 719-725, 1987] ;
      - La Forskoline [voir Ortonne J.P., J. Int. Med Res., 18, 8C-17C, 1990] ;
      - Les bases xanthiques telles que la théophylline et la caféine ;
   f) Les composés qui stimulent l'activité ou l'expression de la tyrosinase par activation de la protéine kinase C :
      - Les diacylglycérols tels que le 1-oleyl 2-acétyl glycérol [voir Allan AE et al, J. Invest. Dermatol., 105, 5, 687-692, 1995 et le document WO-A-98/55085] ;
      - Les psoralènes [voir Anthony F.A. et al, Photodermatol. Photoimmunol. Photomed 1997, 13, 9-16]
3) Les composés qui stimulent le transfert des mélanosomes des mélanocytes vers les kératinocytes par stimulation des récepteurs PAR-2, comme décrits dans le document WO-A-99/04752.
4) Et les mélanges de ces composés.

Selon un mode préféré de réalisation de l'invention, on utilise comme activateurs de mélanogénèse, les extraits de rosacée, notamment les extraits de *Sanguisorba officinalis,* les extraits de chrysanthème, les alpha-hydroxyacides et leurs dérivés, les alpha-cétoacides et leurs dérivés, les béta-hydroxyacides et leurs dérivés, les rétinoïdes et leurs dérivés, l'extrait de Caesapinia sappan L., les extraits de Parameria laevigata, de Piper cubeba, de Sonchus arvensis L., de Pluchea indica L., de Massoia aromatica Becc., d'Alstonia scholaris, d'Alycia reindwartii BI., de Cinnamomum sintoc BI, d'Arctostaphylos, de Chenopodium, de Poterium, de Gautheria, et leurs mélanges.

Les compositions selon l'invention peuvent se présenter sous toutes les formes galéniques classiquement utilisées pour une application topique, et notamment sous forme de solutions aqueuses ou hydroalcooliques, d'émulsions huile-dans-eau (H/E) ou eau-dans-huile (E/H) ou multiple (triple : E/H/E ou H/E/H), de gels aqueux, de produits anhydres déshydratés, ou de dispersions d'une phase huileuse dans une phase aqueuse à l'aide de sphérules, ces sphérules pouvant être des nanoparticules polymériques telles que les nanosphères et les nanocapsules, ou des vésicules lipidiques de type ionique (liposomes) et/ou non ionique. Ces compositions sont préparées selon les méthodes usuelles.

En outre, les compositions selon l'invention peuvent être plus ou moins fluides et avoir l'aspect d'une crème blanche ou légèrement teintée, d'une pommade, d'un lait, d'une lotion, d'un sérum, d'une pâte, d'une mousse. Elles peuvent aussi se présenter sous forme solide, et par exemple sous forme de stick coulé.

Selon un mode préféré de réalisation, la composition est sous forme d'émulsion, et plus particulièrement d'émulsion H/E.

Quand la composition selon l'invention est une émulsion, elle comporte une phase huileuse. Celle-ci contient de préférence au moins une huile, notamment une huile physiologiquement acceptable. Elle peut contenir en outre d'autres corps gras.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam ;
- les alcools gras ayant de 8 à 26 atomes de carbone, comme l'alcool cétylique, l'alcool stéarylique et leurs mélanges (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Les émulsions contiennent généralement au moins un émulsionnant choisi de préférence parmi les émulsionnants amphotères, anioniques, ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres; les tensioactifs siliconés comme les dimethicone copolyols tels que le mélange de cyclomethicone et de dimethicone copolyol, vendu sous les dénominations DC 5225C et DC 3225C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; et leurs mélanges ; les esters d'acides gras et de sorbitan oxyalkylénés comprenant par exemple de 20 à 100 OE, et par exemple ceux commercialisés sous les dénominations commerciales TWEEN par la société Uniqema ; les éthers d'alcools gras oxyalkylénés (oxyéthylénés et/ou oxypropylénés) comme le ceteareth-20 ; les esters de sucres, notamment les esters de sucrose comme le stéarate de sucrose et le tristéarate de sucrose comme le Tegosoft de la société Degussa, et les esters de glucose comme le sesquistéarate de méthylglucose comme le Glucate SS de la société Noveon ; et les mélanges de ces émulsionnants comme par exemple le mélange de mono-stéarate de glycéryle et de stéarate de polyéthylène glycol (100 OE) commercialisé sous la dénomination Simulsol 165 par la société Seppic et le mélange de stéarate de glycéryle et de stéarate de PEG-100, commercialisé sous la dénomination ARLACEL 165 par la société Uniqema.

On peut ajouter à ces émulsionnants, des co-émulsionnants tels que par exemple les alcools gras ayant de 8 à 26 atomes de carbone, comme l'alcool cétylique, l'alcool stéarylique et leurs mélanges (alcool cétéarylique), l'octyl dodécanol, le 2-butyloctanol, le 2-hexyldécanol, le 2-undécylpentadécanol ou l'alcool oléique.

On peut aussi préparer des émulsions sans tensioactifs émulsionnants ou en contenant moins de 0,5 % du poids total de la composition, en utilisant des composés appropriés, par exemple les polymères ayant des propriétés émulsionnantes tels que les polymères carboxyvinyliques modifiés commercialisés sous les dénominations Carbopol 1342 et Pemulen par la société Noveon ; les polymères et copolymères d'acide 2-acrylamido 2-méthylpropane sulfonique (AMPS), éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ou comme le polymère en émulsion commercialisé sous la dénomination Sepigel 305 par la société Seppic (nom INCl: Polyacrylamide / C13-C14 isoparaffine / laureth-7) ou encore les copolymères d'AMPS à chaîne hydrophobe, commercialisés par la société Clariant sous les noms Aristoflex ; les particules de polymères ioniques ou non ioniques, plus particulièrement des particules de polymère anionique comme notamment les polymères d'acide isophtalique ou d'acide sulfoisophtalique, et en particulier les copolymères de phtalate / sulfoisophtalate / glycol (par exemple diéthylèneglycol / Phtalate / isophtalate/1,4-cyclohexane-diméthanol (nom INCI : Diglycol/ CHDM/I sophtalates/ SIP Copolymer) vendus sous les dénominations Eastman AQ polymer (AQ35S, AQ38S, AQ55S, AQ48 Ultra) par la société Eastman Chemical.

On peut aussi préparer des émulsions sans émulsionnants, stabilisées par des particules siliconées ou des particules d'oxyde métallique tels que Ti02.

De façon connue, la composition de l'invention peut contenir également des adjuvants habituels dans le domaine cosmétique ou dermatologique, tels que les gélifiants hydrophiles ou lipophiles, les actifs hydrophiles ou lipophiles, les conservateurs (par exemple phenoxyethanol et parabens), les antioxydants, les solvants, les parfums, les charges, les filtres UV, les bactéricides, les absorbeurs d'odeur, les matières colorantes, les sels, les polymères. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Bien entendu, l'homme de l'art veillera à choisir le ou les éventuels additifs à ajouter à la composition selon l'invention et leurs quantités, de manière telle que les propriétés avantageuses attachées intrinsèquement à la composition conforme à l'invention ne soient pas, ou substantiellement pas, altérées par l'addition envisagée.

Comme charges qui peuvent être utilisées dans la composition de l'invention, on peut citer par exemple, la poudre de silice ; le talc ; les particules de polyamide et notamment celles vendues sous la dénomination ORGASOL par la société Atochem ; les poudres de polyéthylène ; les poudres de matériaux organiques naturels, telles que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Comme polymères, on peut incorporer en particulier dans la composition selon l'invention, un ou plusieurs gélifiants hydrophiles ou lipophiles. Les gélifiants hydrophiles peuvent être choisis par exemple parmi les polymères carboxyvinyliques comme les carbomers commercialisés sous les dénominations Carbopol par la société Noveon ; les polymères carboxyvinyliques modifiés comme ceux commercialisés sous les dénominations Carbopol 1342 et Pemulen par la société Noveon ; les polymères et copolymères d'acide 2-acrylamido 2-méthylpropane sulfonique (AMPS), éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ou comme le polymère en émulsion commercialisé sous la dénomination Sepigel 305 par la société Seppic (nom INCI : Polyacrylamide / C13-C14 isoparaffine / laureth-7) ou encore les copolymères d'AMPS à chaîne hydrophobe, commercialisés par la société Clariant sous les noms Aristoflex ; les polysaccharides tels que les gommes et notamment la gomme de xanthane. Ces gélifiants peuvent être présents en une quantité allant par exemple de 0,001 à 10 % en poids, de préférence de 0,01 à 5 % en poids et mieux de 0,05 à 3 % en poids par rapport au poids total de la composition.

Comme actifs, on peut citer par exemple :
- les agents hydratants tels que par exemple le lactate de sodium; les polyols, tels que la glycérine, le sorbitol, les polyéthylène glycols ; le mannitol ; les acides aminés ; l'acide hyaluronique ; la lanoline ; l'urée et les mélanges contenant de l'urée, tels que le NMF (« Natural Moisturising Factor ») ; la vaseline ; l'acide N-lauroyl pyrrolidone carboxylique et ses sels ; les acides gras essentiels ; les huiles essentielles ; les hydroxyalkyl urées telles que la N-(2-hydroxyéthyl)-urée commercialisée sous la dénomination (Hydrovance de la société National Starch, et leurs mélanges.
- les actifs anti-vieillissement et anti-rides, et plus particulièrement les α-hydroxyacides et notamment les acides dérivés de fruits, comme les acides glycolique, lactique, malique, citrique, tartrique, mandélique, leurs dérivés et leurs mélanges; les β-hydroxy-acides comme l'acide salicylique et ses dérivés tels que l'acide n-octanoyl-5-salicylique ou l'acide n-dodécanoyl-5-salicylique ; les α-céto-acides comme l'acide ascorbique ou vitamine C, et ses dérivés tels que ses sels comme l'ascorbate de sodium, l'ascorbylphosphate de magnésium ou de sodium ; ses esters comme l'acétate d'ascorbyle, le palmitate d'ascorbyle et le propionate d'ascorbyle, ou ses sucres comme l'acide ascorbique glycosylé, et leurs mélanges ; les β-céto-acides ; les rétinoïdes comme le rétinol (vitamine A) et ses esters, le rétinal, l'acide rétinoïque et ses dérivés, ainsi que les rétinoïdes décrits dans les documents FR-A-2,570,377, EP-A-199636, EP-A-325540, EP-A-402072 ; l'adapalène ; les caroténoïdes ; les dérivés C-glycosides comme le C-β-D-xylopyranoside-n-propane-2-one, notamment le C-β-D-xylopyranoside-n-propane-2-one sous forme de solution à 30 % en matière active dans un mélange eau/propylèneglycol (60/40 % en poids) fabriqué par la société CHIMEX sous la dénomination commerciale MEXORYL SBB^{®} ;
- les vitamines, comme les vitamines A et C indiquées ci-dessus, et aussi comme la vitamine E (tocophérol) et ses dérivés ; la vitamine B3 (ou vitamine PP ou niacinamide) et ses dérivés ; la vitamine B5 (ou panthénol sous ses différentes formes : D-panthénol, DL-panthénol), et ses dérivés et analogues, tels que le panthoténate de calcium, la panthétine, la pantothéine, l'éther de éthyl panthényl, l'acide pangamique, la pyridoxine, le pantoyl lactose, et les composés naturels en contenant tels que la gelée royale; la vitamine D et ses analogues tels que ceux décrits dans le document WO-A-00/26167 ; la vitamine F ou ses analogues tels que les mélanges d'acides insaturés possédant au moins une double liaison et notamment les mélanges d'acide linoléique, d'acide linolénique et d'acide arachidonique, ou les composés en contenant ;
- les antibactériens et anti-séborrhéiques tels que l'acide salicylique, le 2,4,4'-trichloro-2'-hydroxy diphényl éther (ou triclosan), le 3,4,4'-trichlorobanilide (ou triclocarban), l'acide azélaïque, le peroxyde de benzoyle et les sels de zinc comme le lactate, le gluconate, le pidolate, le carboxylate, le salicylate et/ou le cystéate de zinc.

Comme exemples de filtres organiques, on peut citer les familles suivantes : les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40 commercialisé par B.A.S.F.) ; les benzylidènes-camphres, en particulier le 3-benzylidène-camphre, l'acide benzylidènecampho-sulfonique, le benzalkoniumméthosulfate de Camphre, le polyacrylamidométhylbenzylidène camphre, l'acide téréphthalylidène di-camphre sulfonique, et le 4-méthylbenzylidène camphre (Eusolex 6300 commercialisé par Merck) ; les benzimidazoles, en particulier le benzimidazilate (Neo Heliopan AP commercialisé par Haarmann et Reimer), ou l'acide phénylbenzimidazole sulfonique (Eusolex 232 commercialisé par Merck) ; les benzotriazoles, en particulier le drométrizole trisiloxane, ou le méthylène bis-benzotriazolyltétraméthylbutylphénol (Tinosorb M commercialisé par Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 commercialisé par B.A.S.F.), l'octylméthoxycinnamate (Parsol MCX commercialisé par Hoffmann La Roche), ou l'octocrylène (Uvinul 539 commercialisé par B.A.S.F.) ; les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB commercialisé par 3V Sigma), l'éthylhexyltriazone (Uvinul T150 commercialisé par B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S commercialisé par Ciba) ; le drometrizole trisiloxane.

La quantité de filtres dépend de l'utilisation finale souhaitée. Elle peut aller par exemple de 1 à 20% en poids et mieux de 2 à 10% en poids par rapport au poids total de la composition.

La composition de l'invention donne un effet immédiat de bonne mine et, en outre, cet effet se prolonge dans le temps. Elle peut constituer notamment une composition cosmétique de soin de la peau, apportant cet effet de bonne mine qui dure dans le temps.

L'invention porte également sur un procédé de traitement cosmétique de la peau pour lui donner un effet de bonne mine, caractérisé en ce qu'il consiste à appliquer sur la peau, une quantité efficace d'une composition telle que décrite ci-dessus. Généralement, la quantité efficace de composition appliquée sur la peau va de 0,5 à 4 mg/cm², mieux de 1 à 3 mg/cm², et encore mieux de 1 à 2 mg/cm .

Les exemples suivants illustrent l'invention sans présenter un caractère limitatif. Les quantités y sont des pourcentages en poids. Les composés sont indiqués en nom INCI ou en noms chimiques selon les cas.

### Exemple 1 : Emulsion H/E

| *Phase huileuse* | | |
|---|---|---|
| Huile d'abricot | 6 | % |
| Cyclohexasiloxane | 10 | % |
| Stearyl alcohol (and) ceteareth 20 (Ritapro 200 de la société RITA) | 2 | % |
| | | |

| *Phase aqueuse* | | |
|---|---|---|
| Methylglucose sesquistearate (Glucate SS) | 2 | % |
| Gomme de xanthane | 0,25 | % |
| Glycérine | 5 | % |
| Conservateurs | 0,6 | % |
| Eau | qsp 100 | % |
| | | |
| Aluminum starch octenylsuccinate (Dry Flo) | 3 | % |
| Pigments encapsulés (Microcapsules Tagra) | 1,5 | % |
| Extrait de Sanguisorba officinalis (Burnet extract Powder de la société Maruzen) | 1 | % |
| DHA | 5 | % |

Mode opératoire : la phase huileuse a été chauffée à environ 80°C jusqu'à dissolution complète, puis elle a été introduite dans la phase aqueuse préalablement portée à la même température pour former une émulsion H/E à laquelle les pigments, puis le dérivé d'amidon, l'extrait de Sanguisorba et la DHA ont été ajoutés.

On a obtenu une émulsion fluide, apte à procurer, lors de l'application sur la peau, un effet immédiat de bonne mine, qui se prolonge dans le temps. L'émulsion a par ailleurs de bonnes propriétés hydratantes.

### Exemple 2 : Emulsion H/E

| *Phase huileuse* | | |
|---|---|---|
| Huile d'abricot | 7 | % |
| Cyclohexasiloxane | 6 | % |
| Caprylic/capric triglyceride | 7 | % |
| Acide stéarique | 1 | % |
| Polysorbate 61 (Tween 61 V) | 1,35 | % |
| | | |

| *Phase aqueuse* | | |
|---|---|---|
| Sucrose tristearate (Tegosoft) | 2 | % |
| Gomme de xanthane | 0,25 | % |
| Carbomer | 0,3 | % |
| Glycérine | 5 | % |
| Propylène glycol | 1 | % |
| Conservateurs | 1,1 | % |
| C-β-D-xylopyranoside-n-propane-2-one (Mexoryl SBB) | 5 | % |
| Eau | qsp100 | % |
| | | |
| Aluminum starch octenylsuccinate (Dry Flo) | 3 | % |
| Erythrulose | 5 | % |
| Extrait de Chrysanthème (*) | 5 | % |
| Pigments encapsulés (Florasomes) | 1 | % |

| | | |
|---|---|---|
| (*) L'extrait de chrysanthème a été obtenu selon le protocole 1 décrit dans EP-A-1014934 : La poudre de chrysanthème a été mélangée à un solvant d'extraction aqueux constitué par du milieu de culture cellulaire DMEM/F 12,3 : 1 vendu par la société Life Technologies, à raison d'une concentration de 5 grammes de poudre sèche pour 100 ml de solvant. Le mélange a été maintenu sous agitation pendant 4 heures à température ambiante. Le mélange a été alors centrifugé à 1000 tours /minute pendant 8 minutes et le surnageant a été prélevé et soumis à deux cycles de centrifugations/prélèvement identiques. Le dernier surnageant prélevé a été filtré sur filtre 0,22 µm de type Millipore en conditions aseptiques afin d'être stérilisé et conservé à une température de 4°C jusqu'à utilisation. | | |

Mode opératoire de préparation de l'exemple : la phase huileuse a été chauffée à environ 80°C jusqu'à dissolution complète, puis elle a été introduite dans la phase aqueuse préalablement portée à la même température pour former une émulsion H/E à laquelle les pigments, puis le dérivé d'amidon, l'érythrulose et l'extrait de chrysanthème ont été ajoutés.

On a obtenu une composition anti-âge, permettant une homogénéisation du teint et particulièrement adaptée aux peaux matures.

### Exemple 3 : Crème de soin (Emulsion H/E)

| *Phase huileuse* | | |
|---|---|---|
| Glycéryle stéarate / PEG-1 00 stéarate (Arlacel 165) | 2,5 | % |
| Polysorbate 60 (Tween 60 V) | 2,5 | % |
| Alcool cétylique | 1 | % |
| Alcool stéarylique | 1 | % |
| Paraffine | 5 | % |
| | | |

| *Phase aqueuse* | | |
|---|---|---|
| Carbomer | 0,3 | % |
| Base (soude) | 0,2 | % |
| Conservateur | 0,2 | % |
| N-(2-hydroxyéthyl)-urée (Hydrovance) | 5 | % |
| Eau | qsp 100 | % |
| | | |
| Fibres de Nylon 66 | 2 | % |
| Extrait de Sanguisorba officinalis (Burnet extract Powder de la Société Maruzen) | 5 | % |
| Pigments encapsulés (Microcapsules Tagra) | 2 | % |

Mode opératoire : la phase huileuse a été chauffée à environ 80°C jusqu'à dissolution complète, puis elle a été introduite dans la phase aqueuse préalablement portée à la même température pour former une émulsion H/E à laquelle les pigments, puis les fibres de nylon et l'extrait de Sanguisorba ont été ajoutés.

On a obtenu une crème de soin de la peau, donnant un aspect hâlé et satiné, et apte à hydrater la peau et à masquer les dyschromies immédiatement et dans le temps.

### Exemple 4 : Crème de soin (Emulsion H/E)

| *Phase huileuse* | | |
|---|---|---|
| Huile d'abricot | 7 | % |
| Cyclohexasiloxane | 6 | % |
| Caprylic/capric triglyceride | 7 | % |
| Acide stéarique | 1 | % |
| Polysorbate 61 (TWEEN 61 V) | 1,35 | % |
| Sucrose tristearate | 2 | % |
| | | |

| *Phase aqueuse* | | |
|---|---|---|
| Gomme de Xanthane | 0,25 | % |
| Aluminium starch octenylsuccinate (Dry-Flo) | 3 | % |
| Carbomer | 0,3 | % |
| Glycérine | 5 | % |
| Propylène glycol | 1 | % |
| Conservateurs | 1,1 | % |
| Erythrulose | 5 | % |
| Extrait de chrysanthème* | 5 | % |
| Pigments encapsulés dans cires d'esters de jojoba | 1 | % |
| Eau | qsp 100 | % |
| (* lyophilisat d'extrait aqueux de feuilles de *Chrysantemum sinensis)* | | |

### Mode opératoire :

La phase huileuse et la phase aqueuse sont chauffées séparément à la température de 80° C. Sous l'agitation de 4000t/mn fournie par un homogénéisateur Moritz de type Turbo Lab 2100, on verse la phase huileuse dans la phase aqueuse et l'on conserve ces conditions d'agitation et de température pendant 30 minutes. Le mélange est alors introduit dans un homogénéisateur haute pression Soavi de type OBL réglé à la pression de 500 bars pour 3 passages consécutifs. Puis on refroidit le produit jusqu'à retour à la température ambiante.

On obtient ainsi une émulsion huile dans eau, dont les globules huileux ont une taille moyenne inférieure à 200 nm et une polydispersité d'indice inférieure à 0,1 telles que mesurées par un granulomètre à laser de type AMTECH BI 90.

## Revendications

1. Composition comprenant des pigments encapsulés et au moins un agent de coloration de la peau choisi parmi les agents autobronzants, les activateurs de mélanogénèse et leurs mélanges.

2. Composition selon la revendication 1, **caractérisée en ce que** les pigments sont choisis parmi les pigments minéraux et les pigments organiques, interférentiels ou non, traités en surface ou non traités, et leurs mélanges.

3. Composition selon la revendication 1 ou 2, **caractérisée en ce que** les pigments sont encapsulés dans des capsules qui sont en un matériau choisi parmi les esters de jojoba, les polymères ou copolymères d'acide acrylique et/ou d'acide méthacrylique, les dérivés de cellulose, et leurs mélanges.

4. Composition selon la revendication précédente, **caractérisée en ce que** les capsules ont une taille allant de 20 à 700 µm.

5. Composition selon l'une quelconque des revendications précédentes, **caractérisée en ce que** les pigments sont présents en une quantité allant de 10 à 80 % en poids par rapport au poids total du pigment encapsulé.

6. Composition selon l'une quelconque des revendications précédentes, **caractérisée en ce qu'**elle contient une quantité de pigments encapsulés allant de 0,01 à 20 % en poids par rapport au poids total de la composition.

7. Composition selon l'une quelconque des revendications précédentes, **caractérisée en ce que** la quantité d' agent(s) de coloration de la peau va de 0,05 à 15 % en poids par rapport au poids total de la composition.

8. Composition selon l'une quelconque des revendications précédentes, **caractérisée en ce que** les agents autobronzants sont des composés mono- ou poly-carbonylés choisis parmi la dihydroxyacétone, l'isatine, l'alloxane, la ninhydrine, le glycéraldéhyde, l'aldéhyde mésotartrique, la glutaraldéhyde, l'érythrulose, les dérivés de pyrazolin-4,5-diones, les dérivés de 4,4-dihydroxypyrazolin-5-ones, et leurs mélanges.

9. Composition selon l'une quelconque des revendications précédentes, **caractérisée en ce que** les agents autobronzants sont choisis parmi la dihydroxyacétone, l'érythrulose et leurs mélanges.

10. Composition selon l'une quelconque des revendications précédentes, **caractérisée en ce que** les activateurs biologiques de la mélanogénèse sont choisis parmi les composés qui stimulent la synthèse de la mélanine, les composés qui stimulent l'activité ou l'expression de la tyrosinase, les composés qui stimulent le transfert des mélanosomes des mélanocytes vers les kératinocytes par stimulation des récepteurs PAR-2, et leurs mélanges.

11. Composition selon l'une quelconque des revendications précédentes, **caractérisée en ce que** les activateurs biologiques de la mélanogénèse sont choisis parmi les extraits de rosacée, les extraits de chrysanthème, les alpha-hydroxyacides et leurs dérivés, les alpha-cétoacides et leurs dérivés, les béta-hydroxyacides et leurs dérivés, les rétinoïdes et leurs dérivés, l'extrait de Caesapinia sappan L., les extraits de Parameria laevigata, de Piper cubeba, de Sonchus arvensis L., de Pluchea indica L., de Massoia aromatica Becc., d'Alstonia scholaris, d'Alycia reindwartii BI., de Cinnamomum sintoc BI, d'Arctostaphylos, de Chenopodium, Poterium, Gautheria, et leurs mélanges.

12. Composition selon l'une quelconque des revendications précédentes, **caractérisée en ce que** la composition est sous la forme d'une émulsion.

13. Composition selon l'une quelconque des revendications précédentes, **caractérisée en ce qu'**elle constitue une composition cosmétique de soin de la peau.

14. Procédé de traitement cosmétique de la peau pour lui donner un effet de bonne mine, **caractérisé en ce qu'**il consiste à appliquer sur la peau, une quantité efficace d'une composition selon l'une quelconque des revendications 1 à 12.
